(19) Europäisches Patentamt
European Patent Office
Office européen des brevets

(11) Numéro de publication: **0 200 628**
**B1**

(12) # FASCICULE DE BREVET EUROPÉEN

(45) Date de publication du fascicule du brevet: **20.07.88**

(51) Int. Cl.⁴: **C 07 C 69/86**, C 07 C 67/52, A 61 K 31/60, C 07 C 127/01

(21) Numéro de dépôt: **86400832.1**

(22) Date de dépôt: **17.04.86**

(54) **Nouveau dérivé de l'acide acétylsalicylique, sa préparation et les compositions pharmaceutiques qui le contiennent.**

(30) Priorité: **19.04.85 FR 8505962**

(43) Date de publication de la demande: **05.11.86 Bulletin 86/45**

(45) Mention de la délivrance du brevet: **20.07.88 Bulletin 88/29**

(84) Etats contractants désignés: **AT BE CH DE FR GB IT LI LU NL SE**

(56) Documents cité:
**DE-A-845 944**
**FR-A-2 492 368**

**Le dossier contient des informations techniques présentées postérieurement au dépôt de la demande et ne figurant pas dans le présent fascicule.**

(73) Titulaire: **RHONE- POULENC SANTE, Les Miroirs 18 Avenue d'Alsace, F-92400 Courbevoie Cédex (FR)**

(72) Inventeur: **Bertrand, Claude, 28 avenue Jean Giono, F-91100 Saint Germain les Corbeil (FR)**
Inventeur: **Wolff, Gérard, 7 rue Jeanne d'Arc, F-94320 Thiais (FR)**

(74) Mandataire: **Pilard, Jacques, RHONE- POULENC RECHERCHES Service Brevets Pharma 25, Quai Paul Doumer, F-92408 Courbevoie Cedex (FR)**

**EP 0 200 628 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention concerne un nouveau complexe hydraté d'acétylsalicylate de magnésium et d'urée de formule:

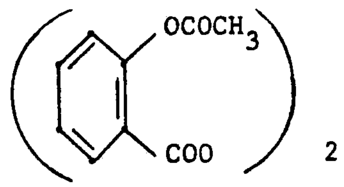 Mg, 2 $H_2NCONH_2$, 2 $H_2O$ (I)

sa préparation et les compositions qui le contiennent

L'acide acétylsalicylique (aspirine) est un médicament qui présente des propriétés analgésiques et antipyrétiques remarquables mais qui, compte tenu de son acidité et de sa faible solubilité, manifeste des effets néfastes sur l'organisme en particulier lorsqu'il est administré par voie orale (mauvaise tolérance gastrique, saveur amère).

Pour éviter ou atténuer ces inconvénients, il a été proposé d'utiliser l'aspirine sous forme de sels afin d'en diminuer l'acidité et d'en améliorer la solubilité. A cet effet ont été proposés des sels de métaux alcalins (sodium) ou alcalino-terreux (calcium, magnésium)

Cependant les divers procédés décrits dans l'art antérieur pour préparer les sels d'aspirine conduisent fréquemment à des produits contenant des impuretés qui proviennent de la dégradation de l'acide acétylsalicylique au cours de la salification ou à des sels hydratés dont la stabilité est médiocre et qui doivent être rapidement déshydratés ; toutefois les sels anhydres obtenus sont souvent hygroscopiques et il est nécessaire de les protéger efficacement de l'humidité lors de leur conditionnement et de leur stockage.

Il a également été proposé d'utiliser des sels plus stables de l'aspirine tels que des sels organiques ou des sels minéraux complexés par des substances organiques. Plus particulièrement ont été proposés des complexes de l'acétylsalicylate de calcium ou de magnésium avec l'urée de formule:

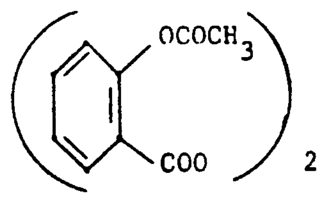 M , $H_2NCONH_2$ (II)

dans laquelle M représente un atome de calcium ou de magnésium.

Selon les procédés connus, par exemple selon le brevet allemand DE 845 944, le complexe acétylsalicylate de calcium-urée est obtenu en traitant, dans un solvant organique, l'acétylsalicylate de calcium par de l'urée.

Selon le brevet français FR 2 492 368, un complexe acétylsalicylate de magnésium-urée serait obtenu après concentration d'une solution d'acétylsalicylate de magnésium et d'urée par évaporation sous pression réduite puis séparation du précipité cristallin obtenu.

Cependant ces complexes, s'ils présentent une meilleure stabilité que les sels métalliques, doivent malgré tout être protégés de l'humidité pour conserver leurs propriétés initiales.

Il a maintenant été trouvé, et c'est ce qui fait l'objet de la présente invention, que le complexe acétylsalicylate de magnésium-urée de formule (I) présente une stabilité nettement supérieure à celle des sels ou des complexes connus.

Plus particulièrement, le nouveau complexe de formule (I), bien qu'il soit hydraté, est plus stable que les sels alcalins ou alcalino-terreux de l'acide acétylsalicylique ou les complexes anhydres tels que le carbasalate de calium.

Le nouveau complexe de formule (I) n'est pas hygroscopique. Il en résulte qu'il n'est pas nécessaire de le déshydrater pour pouvoir le stocker et le transformer en formes pharmaceutiquement acceptables.

Compte tenu de la bonne stabilité de l'eau d'hydratation, il est possible de sécher le produit par application des techniques habituelles sans nuire à la structure du complexe.

Par ailleurs, la présence d'eau ne nuisant pas à la stabilité du complexe, il est possible de lyophiliser ses solutions permettant ainsi d'accéder à de nouvelles formes solubles pour l'administration de l'aspirine que ne permettraient pas de réaliser les hydrates de sels alcalins ou alcalino-terreux.

Selon la présente invention, le nouveau complexe de formule (I) peut être obtenu à partir d'une solution concentrée d'acétylsalicylate de magnésium obtenu in situ par action de l'aspirine sur le carbonate de magnésium dans l'eau en présence d'urée ; le nouveau complexe de formule (I) étant obtenu par cristallisation lente à partir de la solution sursaturée refroidie.

Il est particulièrement avantageux d'opérer à une température comprise entre 15 et 25°C afin d'obtenir une vitesse de réaction suffisante entre l'acide acétylsalicylique et le carbonate de magnésium et d'éviter la désacétylation de l'acide acétylsalicylique.

Pour obtenir le complexe de formule (I), il est nécessaire d'utiliser une quantité d'urée au moins égale à la

quantité stoechiométrique correspondant à la formule (I). De préférence, un produit pur est obtenu lorsque l'on utilise un excès d'urée de 10 à 20 %.

La concentration de la solution du sel magnésien avant la cristallisation doit correspondre à une teneur en aspirine inférieure ou égale à 300 g par kg de solution.

La cristallisation du complexe de formule (I) à partir de sa solution doit être effectuée à une température comprise entre -2 et +2° C. Généralement la cristallisation, effectuée à 0° C, est complète au bout de 24 heures.

Le complexe de formule (I) cristallisé est séparé du mélange réactionnel par filtration ou décantation. Généralement la filtration est préférée car elle permet un meilleur lavage du produit obtenu par un solvant convenable tel que l'acétone.

Le complexe de formule (I) ainsi obtenu peut être séché dans les conditions habituelles, de préférence sous pression réduite, à la température de 20° C, sans subir de dégradation.

L'exemple suivant, donné à titre non limitatif, montre comment l'invention peut être mise en pratique.

**EXEMPLE**

Dans un mèlangeur à poudres, on prépare un mélange homogène de 97,1 g de carbonate basique de magnésium [4 $MgCO_3$, $Mg(OH)_2$, 5 $H_2O$] et de 378 g d'aspirine.

On introduit progressivement en 2 heures le mélange obtenu dans un réacteur de 2 litres, muni d'une agitation, contenant 138,6 g d'urée dans 700 cm3 d'eau à une température comprise entre 20 et 25° C. Le solide se dissout et on observe un dégagement de gaz carbonique. Le pH initial de la solution d'urée est voisin de 7,5 et il se maintient au voisinage de 5,5 - 5,6 au cours de l'addition.

On poursuit l'agitation pendant 30 minutes après la fin de l'addition. La solution est clarifiée par filtration. La solution clarifiée, limpide est refroidie et maintenue à une température comprise entre -2 et +2° C. Cette solution est fortement sursaturée en acétylsalicylate de magnésium. La cristallisation commence au bout d'une à deux heures et se développe lentement. Après 20 heures d'agitation à une température voisine de 0° C, les cristaux obtenus sont séparés par filtration puis lavés sur filtre par 700 cm3 d'acétone et enfin séchés sous pression réduite à une température voisine de 20° C.

On obtient ainsi 223 g de complexe de formule (I) sous forme d'une poudre cristalline blanche.

L'analyse du produit obtenu est la suivante:

| | | | | |
|---|---|---|---|---|
| - Azote | calculé | 11,1 % | trouvé | 11,1 % |
| - Magnésium | calculé | 4,84 % | trouvé | 4,89 % |
| - Aspirine | calculé | 71,3 % | trouvé | 71,4 % |
| - Eau | calculé | 6,7 % | trouvé | 6,8 % |

La teneur en acide salicylique est comprise entre 0,36 et 0,47 %.

La prèsente invention concerne les compositions pharmaceutiques qui contiennent le complexe de formule (I) en association avec un ou plusieurs diluants ou adjuvants compatibles et pharmaceutiquement acceptables.

Comme compositions solides pour administration orale peuvent être utilisés des comprimés, des pilules, des poudres ou des granulés. Dans ces compositions, le produit actif est mélangé à un ou plusieurs diluants inertes tels que l'amidon, la cellulose, le saccharose, le lactose ou la silice. Ces compositions peuvent comprendre également des substances autres que les diluants, par exemple un ou plusieurs lubrifiants tels que le stéarate de magnésium ou le talc, un colorant, un enrobage ou un vernis.

Les compositions solides pour administration orale peuvent se présenter sous forme de lyocs.

Comme compositions liquides pour administration orale, on peut utiliser des émulsions pharmaceutiquement acceptables, des solutions, des suspensions, des sirops ou des élixirs contenant des diluants inertes, tels que l'eau ou l'huile de paraffine. Ces compositions peuvent comprendre des substances autres que les diluants, par exemple des produits mouillants, édulcorants ou aromatisants.

Les compositions pour administration parentérale peuvent être des solutions stériles aqueuses, des suspensions ou des émulsions. Comme véhicule dans ces derniers cas, on peut employer le polyéthylèneglycol, un propylèneglycol, des huiles végétales, en particulier l'huile d'olive, et les esters organiques injectables, par exemple l'oléate d'éthyle.

Ces compositions peuvent contenir également des adjuvants, en particulier des agents mouillants, émulsifiants ou dispersants.

La stérilisation peut se faire de plusieurs façons, par exemple à l'aide d'un filtre bactériologique, en incorporant à la composition des agents stérilisants ou par chauffage. Elles peuvent être également préparées sous forme de compositions solides, rendues stériles par exemple par irradiation, qui peuvent être dissoutes dans de l'eau stérile ou dispersées dans tout autre milieu stérile injectable, éventuellement au moment de l'emploi.

Les compositions pour administration rectale sont des suppositoires qui peuvent contenir, outre le produit actif, des excipients, tels que le beurre de cacao ou un glycéride semisynthétique.

Les compositions selon l'invention sont particulièrement utilisées en thérapeutique humaine comme

analgésiques et antipyrétiques.

Les doses dépendent de l'effet recherché. Elles sont généralement comprises entre 200 et 2000 mg par jour par voie orale pour un adulte en une ou plusieurs prises.

L'exemple suivent, donné à titre non limitatif, illustre une composition selon l'invention.

**EXEMPLE**

On prépare selon la technique habituelle des comprimés ayant la composition suivante :

| | |
|---|---|
| - complexe de formule (I) | 250 mg |
| - amidon | 200 mg |
| - silice colloïdale | 40 mg |
| - stéarate de magnésium | 10 mg |

**Revendications**

pour les Etats contractants: BE,CH,DE,FR,GB,IT,LI,LU,NL,SE.

1. Nouveau complexe hydraté d'acétylsalicylate de magnésium et d'urée caractérisé en ce qu'il répond à la formule :

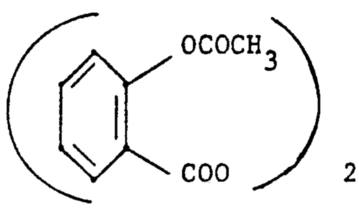

Mg, 2 $H_2NCONH_2$, 2 $H_2O$

2. Procédé de préparation du complexe selon la revendication 1 caractérisé en ce que l'on fait cristalliser lentement le complexe selon la revendication 1 à partir d'une solution sursaturée d'acétylsalicylate de magnésium obtenu in situ par action de l'acide acétylsalicylique sur le carbonate de magnésium dans l'eau en présence d'urée,

3. Procédé selon la revendication 2 caractérisé en ce que la solution du complexe selon la revendication 1 est préparée à une température comprise entre 15 et 25° C.

4. Procédé selon la revendication 2 caractérisé en ce que la cristallisation lente est effectuée à une température comprise entre -2 et +2° C.

5. Procédé selon la revendication 2 caractérisé en ce que l'on utilise une quantité d'urée au moins égale à la quantité stoechiométrique.

6. Composition pharmaceutique caractérisée en ce qu'elle contient le nouveau complexe selon la revendication 1 en association avec un ou plusieurs diluants ou adjuvants compatibles et pharmaceutiquement acceptables.

**Revendications**

pour l'État contractant : AT

1. Procédé de préparation d'un nouveau complexe hydraté d'acétylsalicylate de magnésium et d'urée de formule :

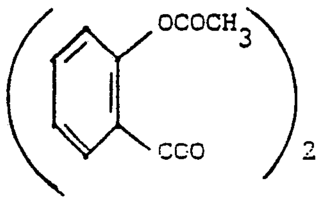

Mg, 2 $H_2NCONH_2$, 2 $H_2O$

caractérisé en ce que l'on fait cristalliser lentement le nouveau complexe à partir d'une solution sursaturée d'acétylsalicylate de magnésium obtenu in situ par action de l'acide acétylsalicylique sur le carbonate de magnésium dans l'eau en présence d'urée.

2. Procédé selon la reveidication 1 caractérisé en ce que la solution du complexe est préparée à une température comprise entre 15 et 25° C.

3. Procédé selon la revendication 1 caractérisé en ce que la cristallisation lente est effectuée à une température comprise entre -2 et +2° C.

4. Procédé selon la revendication 1 caractérisé en ce que l'on utilise une quantité d'urée au moins égale à la quantité stoechiométrique.

**Patentansprüche**

für die Vertragsstaaten : BE,CH,DE,FR,GB,IT,LI,LU,NL,SE.

1. Neuer hydratisierter Komplex von Magnesiumacetylsalicylat und Harnstoff, dadurch gekennzeichnet, daß er der Formel

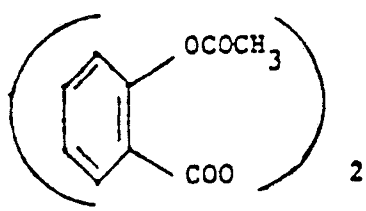

Mg, 2 $H_2NCONH_2$, 2 $H_2O$

entspricht.

2. Verfahren zur Herstellung des Komplexes gemäß Anspruch 1, dadurch gekennzeichnet, daß man den Komplex gemäß Anspruch 1 aus einer übersättigten Lösung von Magnesiumacetylsalicylat, erhalten in situ durch Einwirkung von Acetylsalicylsäure auf Magnesiumcarbonat in Wasser in Gegenwart von Harnstoff, langsam kristallisieren läßt.

3. Verfahren gemäß Anspruch 2, dadurch gekennzeichnet, daß die Lösung des Komplexes gemäß Anspruch 1 bei einer Temperatur zwischen 15 und 25° C hergestellt wird.

4. Verfahren gemäß Anspruch 2, dadurch gekennzeichnet, daß die langsame Kristallisation bei einer Temperatur zwischen -2 und +2° C bewirkt wird.

5. Verfahren gemäß Anspruch 2, dadurch gekennzeichnet, daß man eine Harnstoffmenge verwendet, die mindestens gleich der stöchiometrischen Menge ist.

6. Pharmazeutische Zusammensetzung, dadurch gekennzeichnet, daß sie den neuen Komplex gemäß Anspruch 1 in Assoziation mit einem oder mehreren verträglichen und pharmazeutisch annahmbaren Verdünnungs- oder Hilfsmitteln enthält.

**Patentansprüche**

für den Vertragsstaat AT:

1. Verfahren zur Herstellung eines neuen Hydratkomplexes von Magnesiumacetylsalicylat und Harnstoff der Formel:

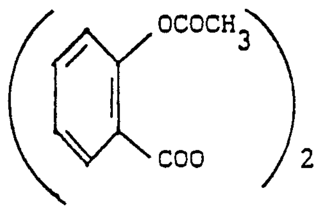 Mg, 2 $H_2NCONH_2$, 2 $H_2O$

dadurch gekennzeichnet, daß man den neuen Komplex langsam aus einer übersättigten Lösung von Magnesiumacetylsalicylat, die in situ durch Einwirkung von Acetylsalicylsäure auf Magnesiumcarbonat in Wasser in Gegenwart von Harnstoff erhalten wurde, kristallisieren läßt.

2. Verfahren nach Anspruch 1, dadurch gekennzeichnet, daß die Lösung des Komplexes bei einer Temperatur zwischen 15 und 25° C hergestellt wird.

3. Verfahren nach Anspruch 1, dadurch gekennzeichnet, daß die langsame Kristallisation bei einer Temperatur zwischen -2 und +2° C erfolgt.

4. Verfahren nach Anspruch 1, dadurch gekennzeichnet, daß man eine der stöchiometrischen Menge zumindest gleiche Menge an Harnstoff verwendet.

**Claims**

for the contracting States : BE,CH,DE,FR,GB,IT,LI,LU,NL,SE.

1. A new hydrated complex of magnesium acetylsalicylate and urea of the formula:

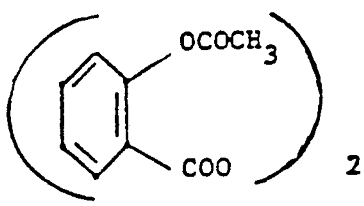 Mg, 2 $H_2NCONH_2$, 2 $H_2O$

2. The process for preparing the complex according to claim 1, wherein the complex according to claim 1 is crystallized slowly from a supersaturated solution of magnesium acetylsalicylate, which is obtained in situ by the action of acetylsalicylic acid on magnesium carbonate in water in the presence of urea.

3. The process according to claim 2, wherein the solution of the complex according to claim 1 is prepared at a temperature of between 15 and 25° C.

4. The process according to claim 2, wherein the slow crystallization is performed at a temperature of between -2 and +2° C.

5. The process according to claim 2, wherein an amount of urea is used which is at least equal to the stoichiometric amount.

6. A pharmaceutical composition which contains the new complex according to claim 1 in combination with one or more diluents or adjuvants which are compatible and pharmaceutically acceptable.

**Claims**

for the contracting State : AT

1. A process for preparing a new, hydrated complex of magnesium acetylsalicylate and urea of the formula:

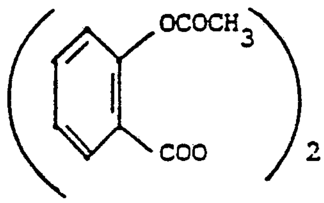 Mg, 2 $H_2NCONH_2$, 2 $H_2O$

wherein the new complex is crystallized slowly from a supersaturated solution of magnesium acetylsalicylate, which is obtained in situ by the action of acetylsalicylic acid on magnesium carbonate in water in the presence of urea.

2. The process according to claim 1, wherein the solution of the complex is prepared at a temperature of between 15 and 25°C.

3. The process according to claim 1, wherein the slow crystallization is performed at a temperature of between -2 and +2°C.

4. The process according to claim 1, wherein an amount of urea is used which is at least equal to the stoichiometric amount.